Europäisches Patentamt

**(19)** European Patent Office

Office européen des brevets

**(11)** **EP 0 662 321 B1**

**(12)** # EUROPEAN PATENT SPECIFICATION

**(45)** Date of publication and mention
of the grant of the patent:
**20.10.1999 Bulletin 1999/42**

**(51)** Int. Cl.$^6$: **A61K 9/00**

**(21)** Application number: **94119482.1**

**(22)** Date of filing: **09.12.1994**

**(54)** **Method for producing osmosis-controlled tablets avoiding the use of chlorinated hydrocarbons**

Verfahren zur Herstellung osmosisgesteuerter Tabletten ohne Verwendung von chlorierten Kohlenwasserstoffen

Méthode de préparation des tablettes à commande d'osmose évitant l'utilisation des hydrocarbures chlorinés

**(84)** Designated Contracting States:
**AT CH DE DK GB LI NL SE**
Designated Extension States:
**LT SI**

**(30)** Priority: **09.12.1993 HU 9303504**

**(43)** Date of publication of application:
**12.07.1995 Bulletin 1995/28**

**(73)** Proprietor:
**EGIS GYOGYSZERGYAR RT.**
**1106 Budapest (HU)**

**(72)** Inventors:
• **Fekete Dr. Pál**
**H-1051 Budapest (HU)**
• **Király, Mária born Ignácz**
**H-1161 Budapest (HU)**
• **Sipos, Gábor**
**H-1162 Budapest (HU)**
• **Jámbor, Zsuzsanna, born Hoffmann**
**H-1182 Budapest (HU)**
• **Ujfalussy, Dr. György**
**H-1093 Budapest (HU)**

• **Gora, Magdolna, born Hernyes**
**H-2117 Isaszeg (HU)**
• **Klebovich, Dr. Imre**
**H-1125 Budapest (HU)**
• **Drabant, Dr. Sándor**
**H-1171 Budapest (HU)**
• **Mándi, Dr. Attila**
**H-1026 Budapest (HU)**
• **Kiss, Gizella, born Szabo**
**H-1136 Budapest (HU)**
• **Bárczay, Erzsébet**
**H-1072 Budapest (HU)**
• **Krisztián, Mária**
**H-1161 Budapest (HU)**

**(74)** Representative:
**Beszédes, Stephan G., Dr.**
**Patentanwalt,**
**Münchener Strasse 80a**
**85221 Dachau (DE)**

**(56)** References cited:
**DE-A- 3 417 113**      **DE-A- 3 438 291**
**US-A- 4 327 725**      **US-A- 4 612 008**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The invention is concerned with novel diffusion-osmotic controlled drug-release one-layer or two-layer pharmaceutical tablet cores and a process for preparing them.

Several osmotic drug-release systems became known in the pharmaceutical technology in the past decades. Thus osmotic compositions prepared according to the first patent specifications (US-PS 3,845,770 and 3,916,899) consisted of the active principle and a semipermeable coat surrounding it. Being permeable to water, the coat makes possible the penetration of water from the environment into the interior of the composition; however, the coat is impermeable to the active principle and therefore, it impedes the molecules of the active principle to get out through the coat into the environment. Thus, the getting-out of the active principle of the composition is provided by one or more hole(s) formed on the coat. During the release of the active principle the composition takes up water from the environment through the coat, then the solution or suspension of the active principle is pressed out into the environment through the opening(s) being present on the coat under effect of the osmotic pressure developing in the system. The rate of water uptake and consequently, the rate of active principle release is determined by the permeability to water of the wall; or, the potential difference between the osmotic pressure, more precisely the difference between the chemical potential of the solution formed within the composition and that of the environment (the decrease of osmotic pressure through the coat or the extent or difference of chemical potentials), respectively.

However, a composition of this type is useful only for water-soluble active principles of for substances slightly soluble in water when those are employed in a mixture with water-soluble, so-called osmotic additives (auxiliaries). An additional drawback of these compositions lies in that, after cessation of the saturation concentration, the osmotic pressure within the composition decreases and therefore, the zero-order, i.e. time-constant rate of active principle release, a great advantage of the composition, is changed.

For eliminating the above drawbacks, a composition has been prepared according to US-PS 4,111,202, which contains two layers instead of one layer having osmotic properties within the semipermeable coat. One layer contains the active principle, optionally in a mixture together with an osmotic additive, whereas the other layer, separated from the first layer by an elastic membrane, comprises only an osmotic additive. A hole is bored only on the outer semipermeable coat comprising both layers, from the side of the layer containing the active principle. During the release of the active principle, water diffuses into both layers through the semipermeable coat; under effect of development of the osmotic pressure, the pressing-out of solution (or suspension) formed in the layer containing the active principle begins through the opening formed in the wall. This layer is widened towards the elastic membrane under effect of the osmotic pressure of additive dissolving within the layer which contains only osmotic additive and is separated from the first layer by the elastic membrane. While widening, this layer pushes the solution or suspension of the active principle forwards and completely presses it out of the chamber part provided with the opening.

Although these compositions have completely met the desired purpose, i.e. the assuring of the release of water-insoluble active principles at a steady rate, they proved to be unsuitable for the practical use. That is, the development of an elastic membrane separating the layer of osmotic additive from the layer of active principle and the sticking of the layer containing the osmotic additive together with the layer comprising the active principle, respectively, required complicated technical solutions making the preparation of these compositions highly expensive.

According to US-PS 4,327,725 which describes an osmotic device for the controlled delivery of a beneficial agent to a fluid environment of use, comprising:

a. a wall formed of a semipermeable material permeable to the passage of an exterior aqueous fluid present in environment of use and substantially impermeable to the passage of beneficial agent, the semipermeable wall surrounding and forming;

b. a compartment containing a layer of beneficial agent, that is insoluble to very soluble in the aqueous fluid and a layer of an expandable hydrogel; and

c. a passageway in the semipermeable wall communicating with the layer of beneficial agent in the compartment and with the exterior of the device
the technical difficulties discussed above can be eliminated by using high molecular crosslinked substances, so-called hydrophilic polymers (without hydroxypropyl-methylcellulose having been named) swelling by water uptake, instead of low molecular osmotic substances in the osmotic layer containing no active principle. That is, in this case the polymeric substance swells with a sharp interface under effect of water uptake and, while pushing forwards the aqueous solution or suspension of the layer containing the active principle, presses it out through the opening being present on the coat. Thus, the elastic membrane between the layers containing active principle and that containing no active principle, respectively, can be omitted at this solution and the two-layer tablet can be prepared on industrial scale by using layer-tabletting equipments known and employed for several decades in the tabletting technology. Subsequently, the two-layer tablets are provided with a semipermeable coat, usually by employing solutions of cellulose esters in methylene chloride/methanol in a manner known from earlier patent specifications and then, a hole of proper size is bored on the coat, on the layer containing the active principle.

In the practice, however, this solution also proved to be unsuitable since, by using semipermeable coats the hydrogels employed were unable to resorb sufficient amount of liquid at an appropriate rate through the coat to swell to such an extent which is required for the complete pressing-out of the aqueous solution or suspension of the active principle being in the other layer through the opening formed on the coat. In order to solve the above problem an osmotic composition was developed wherein the layer containing no active principle comprises also a low molecular osmotic substance in addition to the swelling polymer. Thus, the osmotic pressure of this layer is enhanced and the flowing-in of water is accelerated. This osmotic pharmaceutical composition is disclosed in DE-PS 3,417,113 and US-PS 4,612,008.

The composition according to the above patent specifications is a film-coated two-layer tablet, one layer of which contains active principle, low molecular osmotic substance, high molecular osmotic substance and hydrophilic polymer; and the other layer of which incorporates osmotic hydrophilic polymer and low molecular osmotic substance. For assuring the release of the active principle, a hole is bored on the coat from the side of the layer comprising the active principle.

Although the above composition can successfully be used for the preparation of osmotic, prolonged-action compositions, it is burdened by several unpreferable properties, the elimination of which could mean a substantial progress in the preparation of prolonged-action dosage forms.

Such an unadvantageous property of these pharmaceutical compositions lies in that, beside the hydrophilic polymer, low molecular osmotic substances also should be used in the "placebo" layer for increasing the rate of penetration of water through the semipermeable coat. Conclusively, this leads to an increase in the mass of this layer, and as a final outcome, to an increase in the tablet mass, to an expansion of the physical dimension of the tablet, which may be inconvenient on ingestion of the tablet.

An other common drawback of all osmotic compositions known in the Art appears in that the semipermeable coat is prepared from a solution of the coating material in an organic solvent, namely methylene chloride/methanol. The reason for this lies in that though in the Prior Art many polymers have been enumerated which could be useful to develop the semipermeable coat, however, only various cellulose esters are used in the practice, for the dissolving of which the use of chlorinated solvents is indispensable according to the knowledge in the Art. Thus, for the preparation of osmotic compositions known in the Art, chlorinated solvents are employed which are dangerous to an increased degree both to the health and environment. Although the harmful effects of these can be eliminated by maintaining the appropriate technical measures (laminar-flow work-places, recovery of the solvents), this is accompanied by a considerable increase in the production costs.

An additional drawback of two-layer osmotic compositions lies in that the layer containing the active principle should be bored out. If the so-called "placebo" layer is bored through, the active principle is of course not released; whereas on boring both layers of the tablet, the placebo layer is also pressed out in an aqueous medium therefore, the tablet essentially behaves as a "simple" osmotic system which cannot provide the complete pressing-out of the layer containing the active principle. For this reason, such a technical solving of the preparation of two-layer tablets known in the Art has to be used which provides to distinguish the two parts (halves) of tablet before the boring and insures that only the coat covering the tablet part incorporating the active principle is bored through. Thus, an important progress of manufacturing of the composition would be brought above by developing a composition wherein, by boring out both tablet parts, only the layer containing the active principle is pressed out from the tablet because of the peculiar property of the "osmotic" pressing-out layer. The boring-out on both tablet parts is easy to carry out by suitably directing the way of light in boring systems using laser ray.

Thus the very important problem underlying to the invention is to create a diffusion-osmotic controlled drug-release pharmaceutical one-layer or two-layer pharmaceutical tablet core in which the osmotic additives can be omitted from beside the osmotic polymer in osmotic drug-release systems and by which the necessity of the use of organic solvents of the type of chlorinated solvents during formation of the coats has been eliminated and it has been provided for that, by boring out both parts of the two-layer tablet core, the pressing-out proceeds only from the layer containing the active principle, as well as a process for preparing this.

Surprisingly this has been solved by the invention.

Now surprisingly it has been found that all the three parts of the above problem can be solved by preparing a simultaneously diffusive and osmotic drug-release composition instead of an only osmotic drug-release composition. Thus it has been found that ammonium methacrylate copolymers, till now used as coating materials of diffusion-controlled pharmaceutical compositions according to the known Art (cf. DE 34 38 291 A 1), can be employed for coating the tablets, when hydropropyl-methylcellulose is employed as hydrophilic swelling material. In this way, on the one hand, the release of active principle(s) from the composition by diffusion is insured and, on the other hand, hole(s) is/are bored on the coat whereby it is provided for that, in the interior of the composition the solution or suspension of the active principle(s) is released from the composition also by pressing-out through the hole(s) under the pressure developing due to the swelling of hydroxpropyl-methylcellulose.

Hence the subject-matter of the invention is a diffusion-osmotic controlled drug-release one-layer or two-layer pharmaceutical tablet core containing one or more therapeutically active principle(s) and a hydrophilic polymer and being coated with a polymeric film-coat, in case of a two-layer tablet core containing the active principle(s) and hydrophilic polymer in the first layer thereof and hydrophilic polymer in the second layer thereof, and the tablet core further having at least one bore on the film-coat in contact with the tablet core [in case of one-layer tablet core] or on the part of the film-coat in contact with the first tablet core layer containing the active principle(s) [in case of two-layer tablet core], characterized in that the hydrophilic polymer is hydroxypropyl-methylcellulose and the coating material of the film-coat contains an ammonium methacrylate copolymer or a mixture of such.

Thus according to the invention there is provided for an one-layer or two-layer tablet core superficially coated with a film containing ammonium methacrylate copolymer(s) and containing polymer(s) dissolving in aqueous media, from which the active principle is/are released partly by molecular diffusion through the ammonium methacrylate copolymer wall and partly by pressing out through the hole(s) bored on the coat together with the aqueous dispersion or solution of the polymer(s).

According to an useful embodiment of the invention the tablet core contains also one or more bore(s) on the part of the film-coat in contact with the second tablet core layer containing hydrophilic polymer and no active principle.

Preferably the bore(s) is/are from 0.01 to 1.0 mm$^2$ in cross-section.

Ammonium methacrylate copolymers used in the coating of the diffusion-osmotic drug-release tablet cores have been employed since the middle of fifties in the pharmaceutical industry. Such copolymers are chiefly employed for the preparation of prolonged-action compositions, which are completely insoluble in water but permeable to various extent and provide thereby the diffusion of molecules of the active principle.

Advantageously the ammonium methacrylate copolymer(s) of the film-coat is/are such having ethyl acrylate, methyl methacrylate and (trimethylammonium)-ethyl methacrylate units.

Preferably the ammonium methacrylate copolymer(s) of the film-coat is/are such with a relative molecular weight of 100,000 to 200,000 containing 1 molar part of ethyl acrylate units, 2 molar parts of methyl methacrylate units and 0.1 to 0.2 molar part of (trimethylammonium)-ethyl methacrylate units.

Particularly the mixture of ammonium methacrylate copolymers of the film-coat is such of

a) an ammonium methacrylate copolymer with a relative molecular weight of 150,000, containing 1 molar part of ethyl acrylate, 2 molar parts of methyl methacrylate and 0.1 molar part of (trimethylammonium)-ethyl methacrylate and

b) an ammonium methacrylate copolymer with a relative molecular weight of 150,000 containing 1 molar part of ethyl acrylate, 2 molar parts of methyl methacrylate and 0.2 molar part of (trimethylammonium)-ethyl methacrylate in a weight ratio of 1 : 10 to 10 : 1.

Advantageously the amount of the film-coat containing the ammonium methacrylate copolymer or mixture of such is from 3 to 25 mg/cm$^2$, related to the tablet core surface.

Such copolymers are listed hereinafter.

1.) <u>Ammonium methacrylate copolymer RL (Eudragit RL)</u>

(Ammonio Methacrylate Copolymer, Type A - USP/NF)
(Aminoalkyl Methacrylate Copolymer RL - JSP I)
(Ethyl acrylate)-(methyl methacrylate)-[(trimethylammonio) ethyl methacrylate] (1 : 2 : 0.2) copolymer, molecular weight 150,000.

Commercially available products (Röhm Pharma, Weiterstadt):

Eudragit RL 100: solid polymer
Eudragit RL 12.5: 12.5% solution in solvent
Eudragit RL 30 D: 30% aqueous dispersion

2.) Ammonium methacrylate copolymer RS (Eudragit RS)

(Ammonio Methacrylate Copolymer, Type B - USP/NF)
(Aminoalkyl Methacrylate Copolymer RS - JSP I)
(Ethyl acrylate)-(methyl methacrylate)-[(trimethylammonio) ethyl methacrylate] (1 : 2 : 0.1) copolymer, molecular weight 150,000.

Commercially available products:

Eudragit RS 100:  solid polymer
Eudragit RS 12.5:  12.5% solution in solvent
Eudragit RS 30 D:  30% aqueous dispersion

Films prepared from ammonium methacrylate RL/RS copolymers are insoluble in water and digestive juices but swell and are permeable to the dissolved substances. This means that, when used according to the known Art, the release of the active principle proceeds by diffusion through these films. The sign RL means the property that the film is easily permeable, whereas the sign RS indicates a difficultly permeable film. Between pH 2 and pH 8 the quaternary ammonium groups of the copolymers are fully dissociated; thus, the permeability of the films is independent of the pH-value and can be controlled by varying the mixing ratio of the two polymers. The manufacturer indicates the field of utilization of Eudragit RL/RS copolymers as follows:

-  coating of skeletal tablets for regulating the initial profile of release;
-  coating of pellets, granulates (granular products), crystals;
-  preparation of granulates or pellets

Röhm Pharma GmbH, Weiterstadt, Prospect (Info RL/RSD-1)
Thus, the usability of ammonium methacrylate copolymers has till now been known only for the production of diffusion-controlled, prolonged-action dosage forms; their utilization for preparing diffusion-osmotic drug-release compositions neither has been disclosed in nor suggested by the Prior Art.
An other indispensable component of the drug-release system according to the invention is hydroxypropyl-methylcellulose (HPMC). By using in tablets coated with ammonium methacrylate copolymer film hydroxypropyl-methylcellulose beside the active principle and in the "osmotic" pressing-out layer, the uptake of water through the coat and the swelling pressure or osmotic pressure, respectively, developing within the tablets are capable of providing the release of active principle(s) at an appropriate rate both by diffusion through the film as well as by pressing out through the opening(s) bored in the coat. As known in the Art, ammonium methacrylate copolymer films are permeable to the active principle(s) and other low molecular additive(s), e.g. sodium and potassium chloride, lactose and mannitol, i.e. the so-called osmotic substances, in addition to water. By using these substances together with active principle(s) in the tablets according to the Prior Art contrary to the invention, the osmotic pressure required to release the active principle(s) cannot be insured in the interior of the tablet core since these low molecular osmotic additives rapidly diffuse out through the coat.
Simultaneously, the films containing ammonium methacrylate copolymer(s), being permeable to small molecules, are impermeable to the high molecular hydroxypropyl-methylcelluloses. Thus, their swelling pressure or osmotic pressure, respectively, is decreased only by their discharge through the hole(s) bored on the coat; on the other hand, due to the high gel strength of hydroxypropyl-methylcellulose polymers, it can be achieved by using these polymers alone or in a concentration higher than 30% by weight in the "osmotic" pressing-out tablet part, that the material of the pressing-out layer is not pressed out of the tablet in spite of both tablet parts having been bored out.
Preferably the tablet core according to the invention contains a hydroxypropyl-methylcellulose containing 20 to 30% by weight of methoxy groups and 5 to 12% by weight of hydroxypropoxy groups and having a viscosity of 3 to 100,000 cP in a 2% by weight aqueous solution as hydrophilic polymer.
Preferably, in case of a two-layer tablet core, in the second tablet core layer containing a hydrophilic polymer and no active principle, the tablet core contains as hydrophilic polymer a hydroxypropyl-methylcellulose having a viscosity higher than 1 000 cP in 2% by weight aqueous solution.
According to an advantageous embodiment of the invention the tablet core contains hydroxypropyl-methylcellulose in an amount of 5 to 30% by weight in the one-layer tablet core, related to the weight of the tablet core (on an uncoated basis), or, in case of a two-layer tablet core, in the first layer of it containing the active principle(s), related to the weight of this layer.

According to another advantageous embodiment of the invention, in case of a two-layer tablet core, it contains hydroxypropyl-methylcellulose in an amount of 30 to 100% by weight in the second tablet core layer containing no active principle, related to the weight of this layer.

The expression "hydroxypropyl-methylcellulose" also includes mixtures of different hydroxypropyl-methylcellulose polymers.

Hydroxypropyl-methylcellulose polymers useful for the pharmaceutical tablet cores according to the invention have been used for the preparation of pharmaceutical compositions since the sixties. According to the known Art, these hydrophilic polymers are used for increasing the viscosity of aqueous solutions or suspensions, for preparing hydrophilic skeletal tablets and matrixes, furthermore as binders of traditional tablets and for coating film tablets.

The commercially available hydroxypropyl-methylcellulose polymers differ from each other in the ratio of hydroxypropoxy groups (HPr groups) to methoxy (Me) groups or in the viscosity of their aqueous solutions, i.e. essentially in the molecular weights of the polymers. The quality demands of various hydroxypropyl-methylcellulose sorts useful for the preparation of pharmaceutical compositions have been described in more detail in the prescriptions of the United States Pharmacopoea. The connection between the trade names, pharmacopoeal names and characteristic parameters of various HMPC sorts are summarized in the following Table 1. At present, hydroxypropyl-methylcellulose sorts are in circulation by the American DOW Chemical Co. under the trade name Methocel® and by the Japanese Shin-Etsu Chemical Co. under the trade name Metolose®.

Table 1

| Relations between the names and quality parameters of hydroxypropyl-methylcellulose polymers | | | | | |
|---|---|---|---|---|---|
| Pharmacopoeal Name of hydroxypropyl--methylcellulose USP HPMC | Producer and Trade Name of hydroxypropyl--methylcellulose | | Methoxy content % by weight | Hydroxypropoxy content % by weight | Viscosity of a 2% by weight aqueous solution cP |
| | DOW chem. Methocel® | Shin-Etsu Metolose® | | | |
| 2910 | E 4M | 60SH-4000 | 29 | 10 | 4 000 |
| 2906 | F 4M | 65SH-4000 | 29 | 6 | 4 000 |
| 2208 | K 4M | 90SH-4000 | 22 | 8 | 4 000 |

The rate of hydratation of the polymer is directly proportional and the gel strength of polymer is inversely proportional to the ratio of hydroxypropyl groups introduced into the free hydroxyl groups of the polysaccharide chain of cellulose. Of course, the gel strength is directly proportional also to the molecular weight of the polymer and the value of viscosity measured in 2% by weight aqueous solution. The viscosity of commercially available hydroxypropyl-methylcellulose polymers with the lowest molecular weight is 5 cP (named Methocel® E5) and 3 cP (named Metolose® 60SH-3), respectively, whereas hydroxypropyl-methylcellulose polymers having the highest molecular weight are Methocel® K100M (with a viscosity of 100,000 cP) and Metolose® 60SH 4000, 65SH 4000 and 90SH 4000 (having a viscosity of 4 000 cP each).

In the prolonged-action tablet cores according to the invention any of hydroxypropyl-methylcellulose polymers can be used in the one-layer tablet cores or in the layer containing the active principle(s) of the two-layer tablet cores, whereas in the "osmotic" pressing-out layer of the two-layer tablet cores only the higher molecular weight hydroxypropyl-methylcellulose polymers having a viscosity higher than 1 000 cP in 2% by weight aqueous solution are advantageous.

Advantageously the tablet core according to the invention also contains [a] filler(s) and/or binder(s) and/or lubricant(s).

Preferably it contains as filler(s) cellulose, microcrystalline cellulose, starch, lactose, mannitol and/or secondary calcium phosphate.

It is also preferred that it contains as binder(s) hydroxypropyl-cellulose, methylcellulose, hydroxypropyl-methylcellulose, polyvinylpyrrolidone, ethylcellulose, gelatine, water-soluble starch, polyoxyethylene glycol, polyvinylalcohol and/or polyvinylbutyral.

Moreover it is preferred that it contains as lubricant(s) magnesium stearate, talc, stearic acid, hydrogenated castor oil and/or colloidal silicon dioxide.

According to a preferred embodiment of the invention the tablet core contains

a) up to 90% by weight of filler(s),

b) up to 5% by weight of binder(s) and/or

c) up to 5% by weight of lubricant(s),
in the one-layer tablet core, related to the weight of the tablet core (on an uncoated basis), or, in case of a two-layer tablet core, in the first layer of it containing the active principle(s), related to the weight of this layer.

According to a further special preferred embodiment of the invention the tablet core in case of a two-layer core contains

a) up to 70% by weight of filler(s),

b) up to 5% by weight of binder(s) and/or

c) up to 5% by weight of lubricant(s)
in the second layer containing no active principle, related to the weight of this layer.

According to a particularly preferred embodiment of the invention the tablet core contains

a) the active principle(s),

b) 5 to 30% by weight of hydroxypropyl-methylcellulose,

c) up to 90% by weight of filler(s),

d) up to 5% by weight of binder(s) and

e) up to 5% by weight of lubricant(s),
in the one-layer tablet core, related to the weight of the tablet core (on an uncoated basis), or, in case of a two-layer tablet core, in the first layer of it, i.e. that containing the active principle(s) [a], related to the weight of this layer, and further the tablet core has the film-coat of 3 to 25 mg/cm$^2$ containing the ammonium methacrylate copolymer or mixture of such, related on the surface of the tablet core, on which coat one or more bore(s) of 0.01 to 1.0 mm$^2$ in cross-section is/are present.

According to a further special particularly preferred embodiment of the invention in case of a two-layer tablet core the second layer of it containing no active principle contains

a) 30 to 100% by weight of hydroxypropyl-methylcellulose having a viscosity higher than 1 000 cP in 2% by weight aqueous solution,

b) up to 70% by weight of filler(s),

c) up to 5% by weight of binder(s) and

d) up to 5% by weight of lubricant(s),
related to the total weight of this layer, and the bore(s) is/are present only on the part of the film-coat in contact with the first layer containing the active principle(s) or beyond on this also on the part of the film-coat in contact with the second layer containing hydrophilic polymer and no active principle.

According to a still further special advantageous embodiment of the invention the tablet core contains between its surface and the film-coat an intermediate coat of hydroxypropyl-methylcellulose. Preferably the amount of this intermediate coat is 1 to 5 mg/cm$^2$.

The rate of drug-release from the pharmaceutical tablet cores according to the invention is determined by two factors: the diffusion of active principle(s) through the tablet coat and its pressing-out in the form of a solution or suspension, which proceeds through the hole(s) bored on the coat, under the effect of differential osmotic pressure. The drug-release from the tablet cores becomes steady after a transitional non-steady state, when water molecules diffuse through the coat into the interior of the tablet, dissolve the active principle(s), furthermore swell and dissolve the hydroxypropyl-methylcellulose. In this steady state, the rate of drug-release can be described by the following correlations.

A) In case of one-layer tablet core

Drug-release by diffusion:

$$dm_D/dt = A^*C_t^*k_D/h$$

wherein

$dm_D/dt$      means the rate of drug-release by diffusion ($MT^{-1}$)
$A$      is the surface of tablet core ($L^2$),
$C_t$      is the saturation concentration of the active principle ($M/L^3$),
$k_D$      means the rate constant of diffusion of the active principle ($ML^{-2}T^{-1}$),
$h$      is the thickness of coat ($L$) and
$M, L$ and $T$      are the dimensions of weight, length and time.

Osmotic drug-release:

$$dm_o/dt = dv/dt^*C = A^*C^*\Delta\pi^*k_v/h$$

wherein

$dm_o/dt$      means the rate of osmotic drug-release ($MT^{-1}$),
$dv/dt$      means the rate of water diffusion into the tablet ($MT^{-1}$),
$C$      is the active principle concentration of the liquid being present in the tablet ($ML^{-3}$),
$\Delta\pi$      means the osmotic pressure drop through the tablet coat ($ML^{-1}T^{-2}$),
$k_v$      is the rate coefficient of diffusion of the water ($ML^{-2}T^{-1}$) and
$M, L$ and $T$      are the dimensions of weight, length and time.

The total rate of drug-release from the tablet core is defined by the sum of drug-release by diffusion + drug-release by osmosis:

$$dm/dt = (k_D^*A^*C_t + k_v^*A^*\Delta\pi^*C)/h$$

or, since $C_t = C$ , when the active principle dissolves in the water diffusing through the film coat,

$$dm/dt = (k_D + k_v^*\Delta\pi)A^*C/h$$

wherein

$dm/dt$      is the total rate of drug-release from the tablet ($MT^1$).

B) In case of two-layer tablet core

Drug-release by diffusion:

$$dm_D/dt = A_A^*C_t^*k_D/h$$

wherein

$A_A$      means the surface of tablet part containing the active principle ($L^2$) and the other symbols are as above defined.

Osmotic drug-release:

$$dm_o/dt = (dv_A/dt + dv_p/dt)^*C = (A_A^*\Delta\pi_A + A_p^*\Delta\pi_p)^*C^*k_v/h$$

wherein

$dv_A/dt$ means the rate of diffusion of water into the tablet part containing the active principle ($MT^{-1}$),

$dv_p/dt$ means the rate of water diffusion into the placebo tablet part ($MT^{-1}$),

$A_p$ is the surface of placebo tablet part ($L^2$),

$\pi_A$ means the osmotic pressure drop through the coat of tablet part containing the active principle ($ML^{-1}T^2$),

$\pi_p$ is the osmotic pressure drop through the coat of placebo part ($ML^{-1}T^2$) and the other symbols are as above defined.

The total rate of drug-release from a two-layer tablet core is defined by the sum of drug-release by diffusion + drug-release by osmosis:

$$dm/dt = A_A{}^*C_t{}^*k_D/h + (A_A{}^*\Delta\pi_A + A_p{}^*\Delta\pi_p){}^*C{}^*k_V/h$$

Thus, the drug-release from the tablet cores according to the invention occurs: by molecular diffusion through the intact coat depending on the diffusivity of the active principle(s) through the ammonium methacrylate copolymer film on the one hand; and by the driving force of osmotic and polymer-swelling pressure developing in the tablet interior through the hole(s) bored on the tablet coat, on the other hand. The ratio of the two ways of drug-release is defined by diffusivity of the active principle(s) through the ammonium methacrylate copolymer film. In case e.g. of active principles well-soluble in water (such as salbutamol, metoprolol, bricanyl$^{®}$ or the like), the concentration of solution formed within the tablet provides a significant drug-release by diffusion. The rate of drug-release of such active principles is defined by the totality of diffusion and polymer-swelling processes. Contrary to this, in case of active principles slightly soluble in water (such as nifedipine), the drug-release by diffusion is low due to the low value of active principle concentration developing in the interior of the tablet core; thus, the extent of drug-release from the tablet core is (defined) chiefly by the driving force of pressure arising from the swelling of the polymer.

A subject-matter of the invention is also a process for preparing the one-layer tablet cores according to the invention, characterized in that the therapeutically active principle(s) and the hydroxypropyl-methylcellulose are mixed and the uncoated tablet cores are prepared by compression and the latter are coated with the film-coat containing ammonium methacrylate copolymer(s) and finally one or more bore(s) is/are established on the film coat.

An embodiment of the process according to the invention for preparing the two-layer tablet cores according to the invention is characterized in that the therapeutically active principle(s) and the hydroxypropyl-methylcellulose are mixed and the uncoated tablet cores serving as the first tablet core layer containing the active principle(s) is prepared by compression and either thereon the second tablet core layer consisting of hydroxypropyl-methylcellulose is pressed or this second tablet core layer is developed by pressing on the uncoated tablet core serving as the first tablet core layer containing the active principle(s) a powder or granulate of hydroxypropyl-methylcellulose and the surfaces of the thus obtained two-layer tablet cores are coated with the film-coat containing ammonium methacrylate copolymer(s) and finally one or more bore(s) is/are established on the film coat on the part of the film-coat in contact with the first tablet core layer containing the active principle(s).

Advantageously the therapeutically active principle(s) and the hydroxypropyl-methylcellulose are mixed together with filler(s) and/or binder(s).

Furthermore it is advantageous that the mixture containing the active principle(s) and the hydroxypropyl-methylcellulose is mixed with lubricant(s) directly or after granulating with binder(s).

Moreover advantageously as powder or granulate of hydroxypropyl-methylcellulose such containing also filler(s) and/or binder(s) and/or lubricant(s) is used.

According to an advantageous embodiment of the process according to the invention one or more bore(s) is/are established also on the part of the film-coat in contact with the second tablet core layer containing no active principle.

Conveniently the coating of the uncoated tablet core is carried out with an aqueous dispersion of the ammonium methacrylate copolymer(s) or a solution containing the ammonium methacrylate copolymer(s) in ethanol, isopropanol or acetone or a mixture thereof.

According to preferred embodiments of the process according to the invention one proceeds as follows.

a) One-layer tablet core

For the preparation of an one-layer tablet core, the active principle(s) is/are homogenized with 5 to 30% by weight of hydroxypropyl-methylcellulose polymer in dry form calculated on the weight of the uncoated tablet core, if desired, together with up to 90% by weight of tablet filler(s) {tablet-filling material(s)}, such as cellulose, lactose, mannitol, starch, microcrystalline cellulose and/or calcium phosphate, and, if desired, granulated in a manner known in the practice of tablet preparation. The granulating may be carried out by using any of both the "dry process" or "wet process". In the case of the dry process, the homogenate is mixed with up to 5% by weight of tabletting binding material(s), e.g. polyvinylpyrrolidone, hydroxypropyl-methylcellulose, polyvinylbutyral and/or hydroxypropyl-cellulose, then briquetted or tabletted, ground and sieved to the desired (usually 0.1 to 1.0 mm) size. When wet-granulated, the homogenate is kneaded together with the solution of [a] binding material(s), such as polyvinylpyrrolidone, hydroxypropyl-cellulose, methyl-cellulose, gelatine and/or polyvinyl alcohol, in water or a $C_{1-3}$-alcohol, or the solution of the binder is sprayed onto the fluidized powder mixture in a fluidized-bed granulating equipment. Subsequently, the granulate formed is dried and sieved to the desired size (usually less than 1.0 mm). Then, after adding lubricant(s), e.g. magnesium stearate, stearic acid, colloidal silicon dioxide, hydrogenated castor oil and/or talc, the original homogenate or the granulate prepared therefrom is compressed to biconvex-shaped tablet cores containing the desired amount of active principle(s). The tablet cores are coated with a film containing ammonium methacrylate copolymer(s) in an amount of 3 to 25 mg/cm$^2$, calculated on the surface of the tablet cores, in a manner known in the practice of film-coating. The coating may be carried out by using a solution of ammonium methacrylate copolymers in ethanol, propanol or acetone or any mixture thereof or, preferably an aqueous latex dispersion of the copolymers. The coating equally may be accomplished in a traditional perforated-wall kettle, in a fluidized-bed device or sphere-coating apparatus. Finally, one or more bore(s) of 0.01 to 1 mm$^2$ in cross-section is/are formed on the coat of tablet core obtained in order to insure the osmotic drug-release. The bore(s) may be formed by using a mechanical boring device or a boring equipment using laser ray.

The quality and quantity of the hydroxypropyl-methylcellulose used for the preparation of the tablet cores, the quality of ammonium methacrylate copolymer used for coating the tablet cores, the amount of coat as well as the diameter of the bore(s) to be formed can be determined by a person skilled in the Art, in the knowledge of the dissolution demands relating to the active principle(s) given, by carrying out a few experiments on the basis of recognitions described in the present invention.

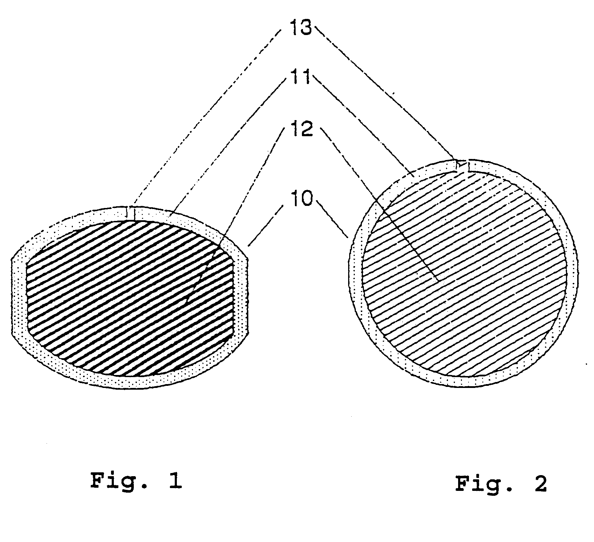

An embodiment of the one-layer tablet cores according to the invention is illustrated by the enclosed Figures 1 and 2,

Figure 1 is the reproduction of the cross-section of a tablet-core after production and

Figure 2 shows the steady state of drug-release after ingestion.

As shown in Figure 2, the ammonium methacrylate copolymer film-coat swelling under influence of water develops a nearly spheric-symmetrical shape on effect of the swelling pressure of hydroxypropyl-methylcellulose polymer being present in the tablet.

The reference numerals used in the Figures 1 and 2 have the following meanings:

10 Diffusion-osmotic drug-release pharmaceutical tablet core.

11 Coat containing the ammonium methacrylate copolymer(s).

12 Tablet core containing active principle and hydroxypropyl-methylcellulose.

13 Opening bored on the coat.

b) Two-layer tablet core

The homogenate or, if desired, the granulate for preparing the layer containing the active principle(s) of two-layer tablet cores are produced as above described for the preparation of one-layer tablet cores. For preparing the other layer of the tablet core containing no active agent, a homogenate is prepared from a 2% by weight solution of hydroxypropyl-methylcellulose having a viscosity higher than 1 000 cP alone or together with up to 70% by weight of filler(s), such as e.g. cellulose, microcrystalline cellulose, lactose, mannitol and/or dicalcium phosphate, which, if desired, are then granulated in a way known in the practice of tabletting, by using any of the processes described above. Subsequently, two-layer tablet cores containing the required amount of active principle(s) in one-layer and the required amount of hydroxypropyl-methylcellulose in the other one, are prepared from the powder mixture of two kinds or, if desired, from the granulates prepared therefrom, to which the lubricant(s) needed had separately been mixed by applying the second tablet core layer to the first one. These operations are carried out in a manner known in the practice of tabletting. For establishing the film-coating, the tablet cores are preferably biconvex-shaped. Then, the tablet cores are coated with a film containing ammonium methacrylate copolymer(s) in an amount of 3 to 25 mg/cm$^2$ calculated on the surface of the two-layer tablet cores. The coating may be carried out by using a solution containing the ammonium methacrylate copolymer(s) in ethanol, propanol or acetone or a mixture thereof or preferably, by employing an aqueous latex dispersion containing the copolymer. (Several instructions relating to the coating process have been published in the booklets of the firm Röhm Pharma Company, the manufacturer of the Eudragit sorts.) Finally, bore(s) of 0.1 to 1.0 mm$^2$ in cross-section are to be formed on the coat of the tablet core established in order to provide the osmotic drug-release.

For functioning of the tablet core it is sufficient to bore one single hole on the coat covering the tablet part containing the active principle(s), though the functioning of the tablet core is not influenced by simultaneously establishing a bore also on the coat covering the other tablet core layer containing no active principle. If desired, several bores may be developed on the tablet core part containing the active principle(s) or on both tablet core parts. The bore(s) may equally be formed by using a mechanical boring device or a boring equipment working with laser ray.

The quality and quantity of the hydroxypropyl-methylcellulose types to be employed in the two tablet core layers, furthermore the quality of the ammonium methacrylate copolymer(s) to be used in the film-coat, the thickness of coat as well as the diameter of the bore(s) can be determined by a person skilled in the Art by carrying out only a few experiments on the basis of the recognitions described in the present invention.

If desired, the tablet cores prepared in this way, which themselves are useful for the therapeutical use, may be provided with an additional water-soluble coat and with an inscription for the sake of identification.

In case it is desired to form a bore only on the tablet core part containing the active principle(s), both tablet core parts should be made distinguishable by colouring one or both granulate(s) or using a compressing device with different shapes; furthermore, when different colours are employed, the coat containing the ammonium methacrylate copolymer(s) controlling the drug-release has to be transparent to make the individual layers (separate layers) recognizable at boring. When bore(s) is/are formed on both parts of the tablet cores, the coat containing the ammonium methacrylate copolymer(s) can also be coloured and in this case, the colouring of tablet cores after boring can be eliminated.

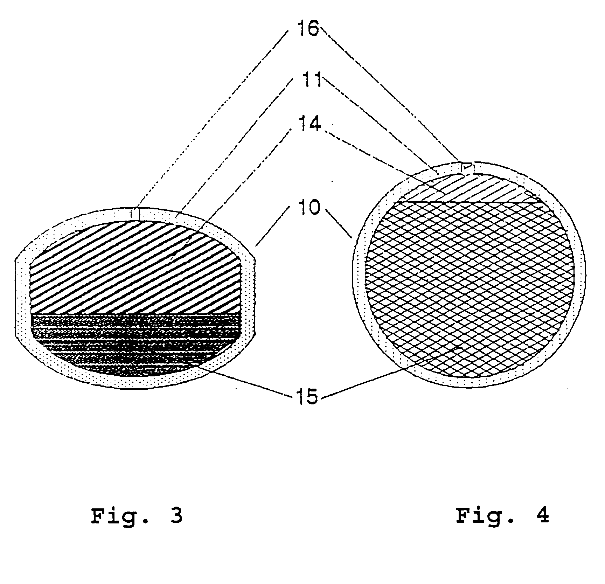

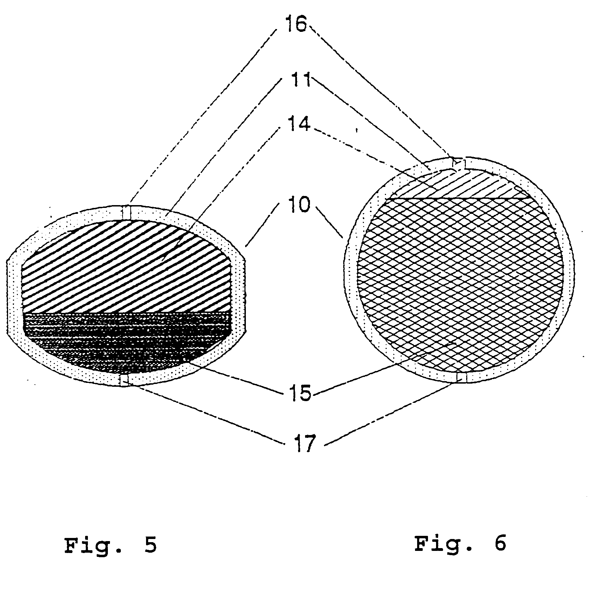

An embodiment of the two-layer tablet cores according to the invention after production is illustrated by the enclosed Figures 3 to 6.

The reference numerals used in the Figures 3 to 6 beyond those already used in Figures 1 and 2 have the following meanings:

14 Diffusion-osmotic drug-release pharmaceutical tablet core part containing the active principle(s) and hydroxypropyl-methylcellulose.

15 Diffusion-osmotic drug-release pharmaceutical tablet core part containing no active principle.

16 Opening on the coat of tablet core part containing the active principle(s).

17 Opening on the coat of tablet core part containing no active principle.

The tablet core according to the invention is useful for controlled release action tablets with a very wide scope of active principles. Its utilization is limited only by possible incompatibility between the active principle(s) and hydroxypropyl-methylcellulose and/or ammonium methacrylate copolymers, respectively; or by the case in which a single dose required exceeds 1 500 mg since the physical size of the tablet prepared in this way makes nearly impossible to ingest it.

The tablet cores according to the invention are particularly preferably useful with active principles for the treatment of chronic diseases.

Examples of active principles of this kind are: $\beta$-adrenergic inhibitors used in heart and circulation diseases such as e.g. propranolol, i.e. chemically 1-{[1'-(methyl)-ethyl]-amino}-3-{naphthyl-1"-oxy}-2-propanol; metoprolol, i.e. 1-[4-(2-methoxyethyl)-phenoxy]-3-[(1-methylethyl)-amino]-2-propanol; oxprenolol, i.e. 1-{[1'-(methyl)-ethyl]-amino}-3-{4"-[2'''-(methoxy)-ethyl]-phenoxy}-2-propanol; pindolol, i.e. 1-{1'H-indol-4'-yloxy}-3-{[1'-(methyl)-ethyl]-amino}-2-propanol; calciumantagonists, such as e.g. nifedipine, i.e. 1,4-di-[hydro-2,6-di-[methyl]-4-[2'-(nitro)-phenyl]-3,5-pyridine dicarboxylic acid dimethyl ester; diltiazem, i.e. (2S-cis)-3-[acetyloxy]-5-[2'-(dimethylamino)-ethyl]-2,3-di-[hydro-2-[4"-(methoxy)-phenyl]-1,5-benzothiazepin-4(5H)-one; verapamil, i.e. $\alpha$-{3-[2'-<3",4"-di-(methoxy)-phenyl>-ethyl-methylamino]-propyl}-$\alpha$-{3''',4'''-di-(methoxy)-phenyl}-$\alpha$-{1''''-(methyl)-ethyl}-acetonitrile; nicardipine, i.e. 1,4-di-[hydro]-2,6-di-[methyl]-4-[3'-(nitro)-phenyl]-3,5-pyridine dicarboxylic acid methyl-2"-[(methyl)-phenylmethyl)-amino]-ethylester; nitrendipine, i.e. 1,4-di-hydro]-2,6-di-[methyl]-4-[3'-(nitro)-phenyl]-3,5-pyridine dicarboxylic acid (ethyl)-(methyl) ester; felodipine, i.e. 1,4-di-[hydro]-4-[2',3'-di-(chloro)-phenyl]-2,6-di-[methyl]-3,5-pyridine dicarboxylic acid (ethyl)-(methyl) ester; gallopamil, i.e. $\alpha$-{3-[2'-<3",4"-di-(methoxy)-phenyl>-ethyl-methylamino]-propyl}-$\alpha$-{3''',4''',5'''-tri-(methoxy)-phenyl}-$\alpha$-{1''''-(methyl)-ethyl}-acetonitrile; the so-called organic nitrate active principles, such as e.g. isosorbide dinitrate, i.e. 1,4 : 3,6-dianhydro-D-glucitol-dinitrate; isosorbide 5-mononitrate, i.e. 1,4 : 3,6-dianhydro-D-glucitol-5-nitrate; nitroglycerol, i.e. 1,2,3-propanetriol-trinitrate; the so-called ACE (angiotensin-converting enzyme) inhibitors, e.g. captopril, i.e. 1-[(2S)-3'-(mercapto)-2'-(methyl)-propionyl]-L-proline; enalapril, i.e. 1-{N-[(S)-1'-(ethoxycarbonyl)-3'-(phenyl)-propyl]-L-alanyl}-L-proline; the so-called central alpha-agonist active principles, e.g. prazosin, i.e. 1-[4'-(amino)-6',7'-di-(methoxy)-quinazolin-2'-yl-]-4-[furan-2''-ylcarbonyl]-piperazine; terazosin, i.e. 1-[4'-(amino)-6',7'-di-(methoxy)-quinazolin-2'-yl]-4-[(tetrahydro-furan-2"-yl)-carbonyl]-piperazine; the so-called lipid-level-lowering active principles, e.g. gemfibrozil, i.e. 5-[2',5'-di-(methyl)-phenoxy]-2,2-di-[methyl]-pentanonic acid; lovastatin, i.e. 1S-[1α(R*),3α,7β,8β(2S*,4S*), 8α,β]-2-methylbutanoic acid 1,2,3,7,8,8a-hexa-[hydro]-3,7-di-[methyl]-8-[2'-<tetrahydro-4'-(hydroxy)-6'-(oxo)-2'H-pyran-2'-yl>-ethyl]-naphth-1-yl ester; the so-called anticoagulant active principles, e.g. ticlopidine, i.e. 5-[<2'-(chloro)-phenyl>-methyl]-4,5,6,7-tetrahydrothieno[3,2-c]pyridine; the so-called vasodilatory active principles, e.g. pentoxifylline, i.e. 3,7-di-[hydro]-3,7-di-[methyl]-1-[5'-(oxo)-hexyl]-1H-purin-2,6-dione; the so-called antiulcer active principles, e.g. cimetidine, i.e. N-[cyano]-N"-{methyl}-N'''-{2-[5'-(methyl)-1H-imidazol-4-yl-methyl-thio]-ethyl}-guanidine; ranitidine, i.e. N-{2'-[5'''-(dimethylamino-methyl)-furan-2'''-yl-methyl-thio]-ethyl}-N''-{methyl}-2-(nitro)-1,1-ethene diamine; the so-called nonsteroidal antiinflammatory agents, e.g. diclofenac, i.e. 2-{[2',6'-di-(chloro)-phenyl]-amino}-phenylacetic acid; naproxen, i.e. (S)-{6-[methoxy]-naphth-2-yl}-α-{methyl}-acetic acid; sulindac, i.e. (Z)-{5-(fluoro)-2-{methyl}-1-{[4'-(methylsulfinyl)-phenyl]-methylene}-1H-indene-3-acetic acid; the so-called antihistamines, e.g. dimetindene, i.e. N,N-di-{methyl}-N-{3-[1'-(pyrid-2'-yl)-ethyl]-1H-indene-2-ethane}-amine; astemizole, i.e. 1-{[4'-(fluoro)-phenyl]-methyl}-2-{N-[1"-[2"'-<4-(methoxy)-phenyl>-ethyl]-piperidin-4"-yl]-amino}-1H-benzimidazole; terfenadine, i.e. α-{4-[1',1'-di-(methyl)-ethyl]-phenyl}-δ-{4"-[hydroxydiphenylmethyl]-piperidin-1"-yl}-butanol; cetirizine, i.e. {2-[4'-[<4"-(chloro)-phenyl>-phenylmethyl]-piperazin-1-yl]-ethoxy acetic acid; the so-called antiasthmatics, e.g. salbutamol = albuterol, i.e. α-{[1',1'-di-(methyl)-ethyl-amino]-methyl}-4-(hydroxy)-m-xylene-α,α'-di-{ol}; terbutaline, i.e. 5-{2'-[1",1"-di-(methyl)-ethyl-amino]-1'-[hydroxy]-ethyl}-1,3-di-(hydroxy)-benzene; theophylline, i.e. 3,7-di-[hydro]-1,3-di-[methyl]-1H-purine-2,6-dione; various analgetic substances, e.g. morphine, i.e. (5α,6α)-7,8-di-[dehydro]-4,5α-[epoxy]-17-[methyl]-morphinane-3,6-diol; codeine, i.e. (5α,6α)-7,8-di-[dehydro]-4,5α-[epoxy]-3-[methoxy]-17-[methyl]-morphinan-6-ol; central nervous system active principles, e.g. amitriptyline, i.e. 5-[3'-<di-(methyl)-amino>-propyliden]-10, 11-di-(hydro)-5H-dibenzo[a,d]cyclohepten; 8-{4'-[4"-(pyrimidin-2'"-yl)-piperazin-1"-yl]-butyl}-8-azaspiro[4,5]-decan-7,9-dion and carbamazepine, i.e. 5H-dibenz[b,f]azepine-5-carboxamide.

The drug-release tablet cores according to the invention can optionally be controlled within an interval from a few hours up to a few days by changing the parameters influencing the rate of release (such as quality and quantity of hydroxypropyl-methylcellulose, quality and thickness of the ammonium methacrylate copolymer film and size and number of bores). However, on the basis of practical points of view it is suitable to adjust the time of release to a duration of 4 to 24 hours.

The invention is illustrated in detail by the following Examples.

Example 1

Round, biconvex-shaped tablet cores of 8 mm in diameter were prepared, which contained 8 mg of salbutamol base <α-{[1',1'-di-(methyl)-ethyl-amino]-methyl}-4-(hydroxy)-m-xylene-α,α'-di-{ol}> as active principle, 160 mg of lactose monohydrate as filler, 6 mg of polyvinylpyrrolidone (Povidon[R] K-90 GAF) as binder, 20 mg of hydroxypropyl-methylcellulose (Methocel[R] K15M, Colorcon) in 2% by weight aqueous solution with a viscosity of 15 000 cP as hydrophilic polymer and 2 mg of magnesium stearate and 4 g of talc as lubricants. The tablets were coated in a traditional dragée kettle with a film containing ammonium methacrylate copolymer using an aqueous coating dispersion with the following ingredients.

| Ingredients of the film | Amount in g | |
|---|---|---|
| | I | II |
| Ammonium methacrylate copolymer RL 30% by weight | 125 | 41.6 |
| Ammonium methacrylate copolymer RS 30% by weight | - | 83.4 |
| Triethyl citrate | 7 | 7 |
| Dimethylpolysiloxane of 35% by weight | 4 | 4 |

The coating of the tablet cores was carried out until achieving a cover of 7 mg/cm$^2$, which means 14.3 mg of coat weight on each tablet. On a part of the coated tablet cores a hole of 0.5 mm diameter was bored, whereafter the rate of active principle release was determined both for the unbored and the bored-out tablet cores by using a paddle-mixer dissolution-investigating equipment at a rate of stirring with 100 revolutions/minute and 900 ml of 0.1 N hydrochloric acid.

The measurement results listed in the following Table 2 were obtained:

Table 2

| Time of dissolution in hours | Sign of the coat | | | |
|---|---|---|---|---|
| | I | | II | |
| | Released amount of the active principle | | | |
| | Unbored | Bored [according to the invention] | Unbored | Bored [according to the invention] |
| 1 hour | 40.4% by weight | 42.2% by weight | 11.4% by weight | 15.1% by weight |
| 2 hours | 54.7% by weight | 66.5% by weight | 20.3% by weight | 26.0% by weight |
| 3 hours | 76.4% by weight | 96.2% by weight | 32.5% by weight | 45.2% by weight |
| 4 hours | 80.1% by weight | 100.0% by weight | 47.1% by weight | 78.6% by weight |
| 5 hours | 95.1% by weight | 100.0% by weight | 58.0% by weight | 87.9% by weight |
| 6 hours | 99.9% by weight | 100.0% by weight | 66.9% by weight | 95.9% by weight |

The following conclusions could be drawn from the above experimental results:

In case of the easily permeable ammonium methacrylate copolymer RL coat, water diffuses in through the coat to the tablet core interior and dissolves salbutamol. The release of salbutamol proceeds partly by diffusion through the coat (see the dissolution data of unbored tablets), partly on effect of the osmotic pressure developing in the tablet core, by pressing out through the bore.

The permeability of the coat can be controlled by mixing the ammonium methacrylate copolymer RL with ammonium methacrylate copolymer RS, however, the film prepared in this manner is not semipermeable since it is permeable also to the active principle in addition to water. An amount of 66% by weight of salbutamol is released by diffusion within 6 hours; whereas additional about 30% by weight of the active principle are pressed out through the opening bored on the coat under effect of the osmotic pressure.

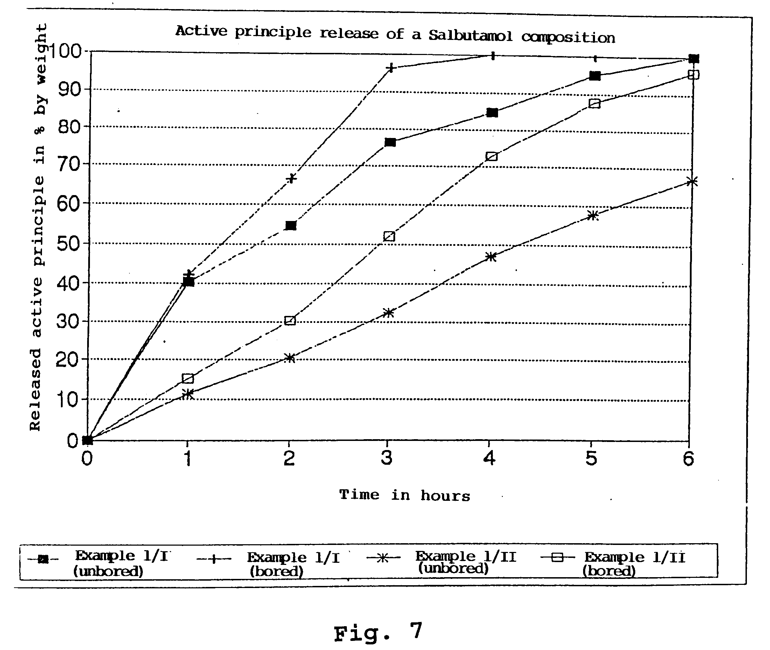

The data of dissolution are graphically illustrated in enclosed Figure 7.

Comparative Test Example

The tablet cores according to Example 1 weighing 200 mg each and containing 8 mg of salbutamol base each as active principle were prepared by omitting the hydrophilic polymer, i.e. 20 mg of hydroxypropyl-methylcellulose from the composition and substituting additional 20 mg of lactose monohydrate therefor. The tablet cores were similarly provided with an amount of 7 mg/cm$^2$ of coat signed as II according to Example 1. Then, after boring a hole of 0.5 mm diameter on a part of the tablet cores, the drug-release of unbored and bored tablet cores was measured.

According to these examinations, 6% by weight of the active principle were released from the unbored tablets, whereas the release was 8% by weight of active principle from the bored tablet cores during 6 hours contrary to the measured value of 66.9% by weight or 95.9% by weight, respectively, by using coat II of Example 1. Thus, it has been proven by this Comparative Test Example that neither diffusive nor osmotic drug-release occurs without the use of hydroxypropyl-methylcellulose as hydrophilic polymer swelling on effect of water.

Example 2

Round, biconvex-shaped tablet cores of 9 mm in diameter were prepared, which contained 30 mg of nifedipine <1,4-di-[hydro]-2,6-di-[methyl]-4-[2'-(nitro)-phenyl]-3,5-pyridine dicarboxylic acid dimethyl ester> as active principle, 211 mg of lactose monohydrate as filler, 30 mg of hydroxypropyl-methylcellulose of 2208 type in a 2% by weight aqueous solution with a viscosity of 15 000 cP as hydrophilic polymer and 1 mg of colloidal silicon dioxide, 2 mg of magnesium stearate and 6 mg of talc as lubricants. The tablet cores were coated by using the following aqueous coating dispersion:

| Ingredients of the coating dispersion | Parts by weight |
|---|---|
| Ammonium methacrylate copolymer RL 30% by weight | 140.0 |
| Triethyl citrate | 8.0 |
| Dimethylpolysiloxane emulsion of 35% by weight | 4.5 |

An amount of 22.3 mg of film-coat were applied onto the tablet cores which corresponds to 7 mg/cm$^2$ of coating material, then a hole of 0.5 mm diameter was bored on the coat.

The drug-release of the tablets was determined by using 0.1 N hydrochloric acid as medium at a temperature of 37°C in a paddle-mixer of 2-type according to USP, at a rate of stirring with 100 revolutions/minute. The amount of active principle released from the tablet cores as a function of the duration of dissolution is listed in the following Table 3:

Table 3

| Time of dissolution in hours | Released amount of the active principle |
|---|---|
| 1 | less than 1% by weight |
| 3 | 5.4% by weight |
| 5 | 25% by weight |
| 8 | 39.5% by weight |
| 10 | 48.3% by weight |
| 24 | 80% by weight |

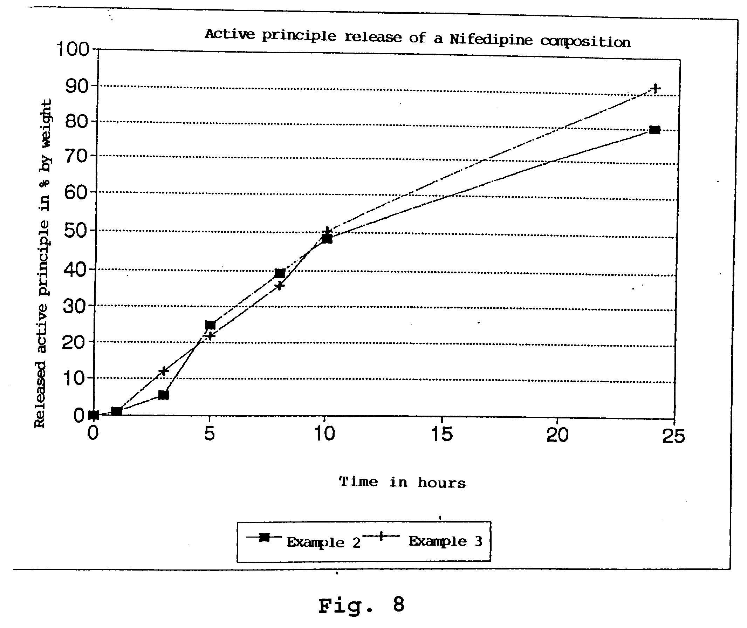

The data of dissolution are graphically illustrated in enclosed Figure 8.

Example 3

Two-layer, round, biconvex-shaped tablet cores of 8 mm in diameter were prepared. One layer of these contained 30 mg of nifedipine as active principle, 142 mg of lactose monohydrate as filler, 8 mg of hydroxypropyl-cellulose as binder, 20 mg of 2208 type hydroxypropyl-methylcellulose having 4 000 cP viscosity in 2% by weight aqueous solution. The tablet cores were coated by using the following coating suspension.

| Ingredients of the coating suspension | Parts by weight |
|---|---|
| Ammonium methacrylate copolymer RL 30% by weight | 42 |
| Ammonium methacrylate copolymer RS 30% by weight | 84 |
| Triethyl citrate | 7 |
| Dimethylpolysiloxane emulsion of 35% by weight | 3 |

The coating of tablet cores was carried out until achieving a coat weight of 28 mg/tablet core = 9.8 mg/cm$^2$. Thereafter, a hole of 0.5 mm diameter was bored on the yellow part containing the active principle of the tablet cores.

The drug-release of the tablet cores was measured by using the method described in Example 2. The results of these measurements are illustrated likewise in Figure 8.

The quantitative data are as listed in the following Table 4.

Table 4

| Time of dissolution in hours | Released amount of the active principle |
|---|---|
| 1 | less than 1% by weight |
| 3 | 12% by weight |
| 5 | 22% by weight |
| 8 | 36% by weight |
| 12 | 54% by weight |
| 24 | 92% by weight |

Example 4

A two-layer tablet core according to Example 3 was prepared in such a manner that the ingredients of the layer containing the active principle were completely identical, whereas the layer containing no active agent contained: 25 mg of 2906 type hydroxypropyl-methylcellulose having 4 000 cP viscosity in 2% by weight aqueous solution as hydrophilic polymer, 25 mg of microcrystalline cellulose as water-insoluble filler, 1,8 mg of polyoxyethylene glycol 6 000 as binder and 0.2 mg of magnesium stearate as lubricant. Subsequently, the two-layer tablet cores were coated until achieving a coat weight of 4 mg each by using an aqueous solution of 2910 type hydroxypropyl-methylcellulose having 6 cP viscosity in 2% by weight aqueous solution and then achieving an additional coat weight of 28 mg = 9.8 mg/cm$^2$ by using a coating dispersion of the following ingredients.

| Ingredients of the coating dispersion | Parts by weight |
|---|---|
| Ammonium methacrylate copolymer RL 30% by weight | 105 |
| Ammonium methacrylate copolymer RS 30% by weight | 105 |
| Triethyl citrate | 12 |
| Dimethylpolysiloxane emulsion of 35% by weight | 6.7 |
| Water | filled up to 350 |

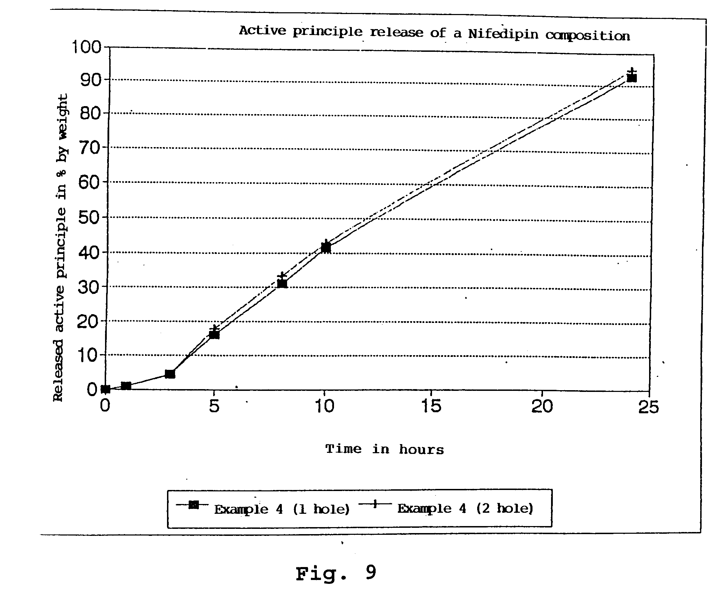

In a part of the tablet cores one hole of 0.3 mm diameter was bored on the tablet core side containing the active principle and in another part of the tablet cores two holes were bored on both sides. The drug-release of tablet cores was determined as described in Example 2. The experimental results are illustrated on the curves of Figure 9.

Example 5

Two-layer tablet cores were prepared by producing tablet cores with the double amount, i.e. 60 mg of active principle from the granulates described in Example 4. In this case the diameter of the tablet cores was 10 mm, whereas their weight was twice larger than that of the tablet cores according to Example 4, i.e. 520 mg. Then, the two-layer tablet cores were coated until achieving a coat weight of 4 mg each by using an aqueous solution of 2910 type hydroxypropyl-methylcellulose having 6 cP viscosity in 2% by weight aqueous solution and successively until achieving additional 28 mg of coat weight = 9.8 mg/cm$^2$ by using a coating dispersion of the following ingredients.

| Ingredients of the coating dispersion | Parts by weight |
|---|---|
| Ammonium methacrylate copolymer RL 30% by weight | 105 |

(continued)

| Ingredients of the coating dispersion | Parts by weight |
|---|---|
| Ammonium methacrylate copolymer RS 30% by weight | 105 |
| Triethyl citrate | 12 |
| Dimethylpolysiloxane emulsion of 35% by weight | 6.7 |
| Water | filled up to 350 |

In a part of the tablet cores one hole of 0.3 mm diameter each was bored on the side containing the active principle and in another part of the tablet cores two holes were bored on both sides. The drug-release of the tablet cores was determined as described in Example 2. The dissolution data listed in the following Table 5 were obtained.

Table 5

| Dissolution time in hours | Released amount of the active principle |
|---|---|
| 3 | 1.7% by weight |
| 5 | 12.4% by weight |
| 8 | 20.7% by weight |
| 24 | 94.9% by weight |

## Claims

1. Diffusion-osmotic controlled drug-release one-layer or two-layer pharmaceutical tablet core (10) containing one or more therapeutically active principle(s) and a hydrophilic polymer and being coated with a polymeric film-coat (11), in case of a two-layer tablet core containing the active principle(s) and hydrophilic polymer in the first layer (14) thereof and hydrophilic polymer in the second layer (15) thereof, and the tablet core (10) further having at least one bore (13; 16) on the film-coat (11) in contact with the tablet core (10) [in case of one-layer tablet core] or on the part of the film-coat (11) in contact with the first tablet core layer (14) containing the active principle(s) [in case of two-layer tablet core], characterized in that the hydrophilic polymer is hydroxypropyl-methylcellulose and the coating material of the film-coat (11) contains an ammonium methacrylate copolymer or a mixture of such.

2. Tablet core as claimed in claim 1, characterized in that it contains also one or more bore(s) (17) on the part of the film-coat (11) in contact with the second tablet core layer (15) containing hydrophilic polymer and no active principle.

3. Tablet core as claimed in claim 1 or 2, characterized in that the bore(s) (13; 16, 17) is/are from 0.01 to 1.0 mm$^2$ in cross-section.

4. Tablet core as claimed in claim 1 to 3, characterized in that as hydrophilic polymer it contains a hydroxypropyl-methylcellulose containing 20 to 30% by weight of methoxy groups and 5 to 12% by weight of hydroxypropoxy groups and having a viscosity of 3 to 100,000 cP in a 2% by weight aqueous solution.

5. Tablet core as claimed in claims 1 to 4, characterized in that, in case of a two-layer tablet core, in the second tablet core layer (15) containing a hydrophilic polymer and no active principle, it contains as hydrophilic polymer a hydroxypropyl-methylcellulose having a viscosity higher than 1 000 cP in 2% by weight aqueous solution.

6. Tablet core as claimed in claims 1 to 5, characterized in that it contains hydroxypropyl-methylcellulose in an amount of 5 to 30% by weight in the one-layer tablet core, related to the weight of the tablet core (on an uncoated basis), or, in case of a two-layer tablet core, in the first layer (14) of it containing the active principle(s), related to the weight of this layer (14).

7. Tablet core as claimed in claims 1 to 6, characterized in that, in case of a two-layer tablet core, it contains hydroxypropyl-methylcellulose in an amount of 30 to 100% by weight in the second tablet core layer (15) containing no active principle, related to the weight of this layer (15).

8. Tablet core as claimed in claims 1 to 7, characterized in that the ammonium methacrylate copolymer(s) of the film-coat (11) is/are such having ethyl acrylate, methyl methacrylate and (trimethylammonium)-ethyl methacrylate units.

9. Tablet core as claimed in claims 1 to 8, characterized in that the ammonium methacrylate copolymer(s) of the film-coat (11) is/are such with a relative molecular weight of 100,000 to 200,000, containing 1 molar part of ethyl acrylate units, 2 molar parts of methyl methacrylate units and 0.1 to 0.2 molar part of (trimethylammonium)-ethyl methacrylate units.

10. Tablet core as claimed in claims 1 to 9, characterized in that the mixture of ammonium methacrylate copolymers of the film-coat (11) is such of

a) an ammonium methacrylate copolymer with a relative molecular weight of 150,000, containing 1 molar part of ethyl acrylate, 2 molar parts of methyl methacrylate and 0.1 molar part of (trimethylammonium)-ethyl methacrylate, and
b) an ammonium methacrylate copolymer with a relative molecular weight of 150.000 containing 1 molar part of ethyl acrylate, 2 molar parts of methyl methacrylate and 0.2 molar part of (trimethylammonium)-ethyl methacrylate
in a weight ratio of 1 : 10 to 10 : 1.

11. Tablet core as claimed in claims 1 to 10, characterized in that the amount of the film-coat (11) containing the ammonium methacrylate copolymer or mixture of such is from 3 to 25 mg/cm$^2$, related to the tablet core surface.

12. Tablet core as claimed in claims 1 to 11, characterized in that it also contains [a] filler(s) and/or binder(s) and/or lubricant(s).

13. Tablet core as claimed in claims 1 to 12, characterized in that it contains as filler(s) cellulose, microcrystalline cellulose, starch, lactose, mannitol and/or secondary calcium phosphate.

14. Tablet core as claimed in claims 1 to 13, characterized in that it contains as binder(s) hydroxypropyl-cellulose, methylcellulose, hydroxypropyl-methylcellulose, polyvinylpyrrolidone, ethylcellulose, gelatine, water-soluble starch, polyoxyethylene glycol, polyvinylalcohol and/or polyvinylbutyral.

15. Tablet core as claimed in claims 1 to 14, characterized in that it contains as lubricant(s) magnesium stearate, talc, stearic acid, hydrogenated castor oil and/or colloidal silicon dioxide.

16. Tablet core as claimed in claims 1 to 15, characterized in that it contains

a) up to 90% by weight of filler(s),
b) up to 5% by weight of binder(s) and/or
c) up to 5% by weight of lubricant(s),
in the one-layer tablet core, related to the weight of the tablet core (on an uncoated basis), or, in case of a two-layer tablet core, in the first layer (14) of it containing the active principle(s), related to the weight of this layer (14).

17. Tablet core as claimed in claims 1 to 16, characterized in that in case of a two-layer core it contains

a) up to 70% by weight of filler(s),
b) up to 5% by weight of binder(s)
and/or
c) up to 5% by weight of lubricant(s)
in the second layer (15) containing no active principle, related to the weight of this layer (15).

18. Tablet core as claimed in claims 1 to 17, characterized in that it contains

a) the active principle(s),

b) 5 to 30% by weight of hydroxypropyl-methylcellulose,

c) up to 90% by weight of filler(s),

d) up to 5% by weight of binder(s) and

e) up to 5% by weight of lubricant(s)

in the one-layer tablet core, related to the weight of the tablet core (on an uncoated basis), or, in case of a two-layer tablet core, in the first layer (14) of it, i.e. that containing the active principle(s) [a], related to the weight of this layer (14), and further the tablet core has the film-coat of 3 to 25 $mg/cm^2$ containing the ammonium methacrylate copolymer or mixture of such related on the surface of the tablet core, on which coat one or more bore(s) (13; 16, 17) of 0.01 to 1.0 $mm^2$ in cross-section is/are present.

19. Tablet core as claimed in claim 18, characterized in that in case of a two-layer tablet core the second layer (15) of it containing no active principle contains

a) 30 to 100% by weight of hydroxypropyl-methylcellulose having a viscosity higher than 1 000 cP in 2% by weight aqueous solution,

b) up to 70% by weight of filler(s),

c) up to 5% by weight of binder(s) and

d) up to 5% by weight of lubricant(s),

related to the total weight of this layer (15), and the bore(s) (16; 17) is/are present only on the part of the film-coat (11) in contact with the first layer (14) containing the active principle(s) [bore(s) 16] or beyond on this also on the part of the film-coat (11) in contact with the second layer (15) containing hydrophilic polymer and no active principle [bore(s) 17].

20. Process for preparing the one-layer tablet cores as claimed in claims 1 to 4, 6, 8, 10, 16 or 18, characterized in that the therapeutical active principle(s) and the hydroxypropyl-methylcellulose are mixed and the uncoated tablet cores (12) are prepared by compression and the latter (12) are coated with the film-coat (11) containing ammonium methacrylate copolymer(s) and finally one or more bore(s) (13) is/are established on the film coat (11).

21. Process as claimed in claim 20 for preparing the two-layer tablet cores as claimed in claims 1, 2, 5, 7, 17 or 19, characterized in that the therapeutical active principle(s) and the hydroxypropyl-methylcellulose are mixed and the uncoated tablet cores (12) serving as the first tablet core layer (14) containing the active principle(s) is prepared by compression and either thereon the second tablet core layer (15) consisting of hydroxypropyl-methylcellulose is pressed or this second tablet core layer (15) is developed by pressing on the uncoated tablet core serving as the first tablet core layer (14) containing the active principle(s) a powder or granulate of hydroxypropyl-methylcellulose and the surfaces of the thus obtained two-layer tablet cores are coated with the film-coat (11) containing ammonium methacrylate copolymer(s) and finally one or more bore(s) (16) is/are established on the film-coat (11) on the part of the film-coat (11) in contact with the first tablet core layer (14) containing the active principle(s).

22. Process as claimed in claim 20 or 21, characterized in that the therapeutically active principle(s) and the hydroxypropyl-methylcellulose are mixed together with filler(s) and/or binder(s).

23. Process as claimed in claims 20 to 22, characterized in that the mixture containing the active principle(s) and the hydroxypropyl-methylcellulose is mixed with lubricant(s) directly or after granulating with binder(s).

24. Process as claimed in claims 20 to 23, characterized in that as powder or granulate of hydroxypropyl-methylcellulose such containing also filler(s) and/or binder(s) and/or lubricant(s) is used.

25. Process as claimed in claims 20 to 24, characterized in that one or more bore(s) (17) is/are established also on the part of the film-coat (11) in contact with the second tablet core layer (15) containing no active principle.

26. Process as claimed in claims 20 to 25, characterized in that the coating of the uncoated tablet core is carried out with an aqueous dispersion of the ammonium methacrylate copolymer(s) or a solution containing the ammonium methacrylate copolymer(s) in ethanol, isopropanol or acetone or a mixture thereof.

**Patentansprüche**

1. Pharmazeutischer diffusions-osmotisch regulierter Arzneimitteltreisetzungs-Einschicht- oder Zweischicht-Tablettenkern (10), enthaltend einen oder mehrere therapeutisch wirksame(n) Haupt- bzw. Grundbestandteil(e) und ein hydrophiles Polymer, und beschichtet mit einem polymeren Filmüberzug (11), im Falle eines Zweischicht-Tablettenkerns enthaltend den(die) wirksame(n) Hauptbestandteil(e) und hydrophiles Polymer in seiner ersten Schicht (14) und hydrophiles Polymer in seiner zweiten Schicht (15), und wobei der Tablettenkern (10) ferner mindestens eine Bohrung (13; 16) auf dem Filmüberzug (11) in Kontakt mit dem Tablettenkern (10) [im Fall eines Einschicht-Tablettenkerns] oder auf dem Teil des Filmüberzugs (11) in Kontakt mit der ersten Tablettenkernschicht (14), welche den(die) wirksame(n) Hauptbestandteil(e) enthält [im Fall eines Zweischicht-Tablettenkerns], aufweist, dadurch gekennzeichnet, daß das hydrophile Polymer Hydroxypropylmethylcellulose ist und das Beschichtungsmaterial des Filmüberzugs (11) ein Ammoniummethacrylat-Copolymer oder eine Mischung davon enthält.

2. Tablettenkern, wie beansprucht in Anspruch 1, dadurch gekennzeichnet, daß er auch eine oder mehrere Bohrung(en) (17) auf dem Teil des Filmuberzugs (11) enthält, der in Kontakt mit der zweiten Tablettenkernschicht (15) ist, welche hydrophiles Polymer und keinen wirksamen Hauptbestandteil enthält.

3. Tablettenkern, wie beansprucht in Anspruch 1 oder 2, dadurch gekennzeichnet, daß die Bohrung(en) (13; 16, 17) einen Querschnitt von 0,01 bis 1,0 $mm^2$ aufweist/aufweisen.

4. Tablettenkern, wie beansprucht in Anspruch 1 bis 3, dadurch gekennzeichnet, daß er als hydrophiles Polymer eine Hydroxypropylmethylcellulose mit 20 bis 30 Gew.-% Methoxygruppen und 5 bis 12 Gew.-% Hydroxypropoxygruppen enthält und eine Viskosität von 3 bis 100 000 cP in einer 2 Gew.-%igen wäßrigen Lösung aufweist.

5. Tablettenkern, wie beansprucht in den Ansprüchen 1 bis 4, dadurch gekennzeichnet, daß im Fall eines Zweischicht-Tablettenkerns selbiger in der zweiten Tablettenkernschicht (15), die ein hydrophiles Polymer und keinen wirksamen Hauptbestandteil enthält, als hydrophiles Polymer eine Hydroxypropylmethylcellulose enthält, die eine höhere Viskosität als 1000 cP in 2 Gew.-%iger wäßriger Lösung aufweist.

6. Tablettenkern, wie beansprucht in den Ansprüchen 1 bis 5, dadurch gekennzeichnet, daß er Hydroxypropylmethylcellulose in einer Menge von 5 bis 30 Gew.-% in dem Einschicht-Tablettenkern, bezogen auf das Gewicht des Tablettenkerns (auf einer nicht-beschichteten Basis), oder, im Falle eines Zweischicht-Tablettenkerns, in der ersten Schicht (14) desselben mit dem(den) wirksamen Hauptbestandteil(en), bezogen auf das Gewicht dieser Schicht (14), enthält.

7. Tablettenkern, wie beansprucht in den Ansprüchen 1 bis 6, dadurch gekennzeichnet, daß im Fall eines Zweischicht-Tablettenkerns, selbiger Hydroxypropylmethylcellulose in einer Menge von 30 bis 100 Gew.-% in der zweiten Tablettenkernschicht (15), welche keinen wirksamen Hauptbestandteil enthält, bezogen auf das Gewicht dieser Schicht (15), enthält.

8. Tablettenkern, wie beansprucht in den Ansprüchen 1 bis 7, dadurch gekennzeichnet, daß das(die) Ammoniummethacrylat-Copolymer(e) des Filmüberzugs (11) ein solches mit Ethylacrylat-, Methylmethacrylat- und (Trimethylammonium)ethylmethacrylat-Einheiten ist/sind.

9. Tablettenkern, wie beansprucht in den Ansprüchen 1 bis 8, dadurch gekennzeichnet, daß das(die) Ammoniummethacrylat-Copolymer(e) des Filmüberzugs (11) ein solches mit einem relativen Molekulargewicht von 100 000 bis 200 000, enthaltend 1 Mol-Teil Ethylacrylat-Einheiten, 2 Mol-Teile Methylmethacrylat-Einheiten und 0,1 bis 0,2 Mol-Teile (Trimethylammonium)ethylmethacrylat-Einheiten, ist/sind.

10. Tablettenkern, wie beansprucht in den Ansprüchen 1 bis 9, dadurch gekennzeichnet, daß die Mischung von Ammoniummethacrylat-Copolymeren des Filmüberzugs (11) ein solche aus

    a) einem Ammoniummethacrylat-Copolymer mit einem relativen Molekulargewicht von 150 000, enthaltend 1 Mol-Teil Ethylacrylat, 2 Mol-Teile Methylmethacrylat und 0,1 Mol-Teile (Trimethylammonium)ethylmethacrylat, und
    b) einem Ammoniummethacrylat-Copolymer mit einem relativen Molekulargewicht von 150 000, enthaltend 1 Mol-Teil Ethylacrylat, 2 Mol-Teile Methylmethacrylat und 0,2 Mol-Teile (Trimethylammonium)ethylmethacrylat, in einem Gewichtsverhältnis von 1:10 bis 10:1 ist.

11. Tablettenkern, wie beansprucht in den Ansprüchen 1 bis 10, dadurch gekennzeichnet, daß die Menge des Filmüberzugs (11) mit dem Ammoniummethacrylat-Copolymer oder einem Gemisch davon von 3 bis 25 $mg/cm^2$

beträgt, bezogen auf die Tablettenkern-Oberfläche.

12. Tablettenkern, wie beansprucht in den Ansprüchen 1 bis 11, dadurch gekennzeichnet, daß er auch [einen] Füllstoff(e) und/oder Bindemittel und/oder Gleitmittel enthält.

13. Tablettenkern, wie beansprucht in den Ansprüchen 1 bis 12, dadurch gekennzeichnet, daß er als Füllstoff(e) Cellulose, mikrokristalline Cellulose, Stärke, Lactose, Mannitol und/oder sekundäres Calciumphosphat enthält.

14. Tablettenkern, wie beansprucht in den Ansprüchen 1 bis 13, dadurch gekennzeichnet, daß er als (ein) Bindemittel Hydroxypropylcellulose, Methylcellulose, Hydroxypropylmethylcellulose, Polyvinylpyrrolidon, Ethylcellulose, Gelatine, wasserlösliche Stärke, Polyoxyethylenglykol, Polyvinylalkohol und/oder Polyvinylbutyral enthält.

15. Tablettenkern, wie beansprucht in den Ansprüchen 1 bis 14, dadurch gekennzeichnet, daß er als (ein) Gleitmittel Magnesiumstearat, Talkum, Stearinsäure, gehärtetes Kastoröl und/oder kolloidales Siliciumdioxid enthält.

16. Tablettenkern, wie beansprucht in den Ansprüchen 1 bis 15, dadurch gekennzeichnet, daß er

   a) bis zu 90 Gew.-% Füllstoff(e),

   b) bis zu 5 Gew.-% (eines) Bindemittel(s) und/oder

   c) bis zu 5 Gew.-% (eines) Gleitmittel(s),
   in dem Einschichttablettenkern, bezogen auf das Gewicht des Tablettenkerns (auf einer nicht-beschichteten Basis), oder, im Falle eines Zweischicht-Tablettenkerns, in der ersten Schicht (14) desselben mit dem(den) wirksamen Hauptbestandteil(en), bezogen auf das Gewicht dieser Schicht (14), enthält.

17. Tablettenkern, wie beansprucht in den Ansprüchen 1 bis 16, dadurch gekennzeichnet, daß, im Fall eines Zweischichtkerns, er

   a) bis zu 70 Gew.-% Füllstoff(e),

   b) bis zu 5 Gew.-% (eines) Bindemittel(s) und/oder

   c) bis zu 5 Gew.-% (eines) Gleitmittel(s),
   in der zweiten Tablettenkernschicht (15), welche keinen wirksamen Hauptbestandteil enthält, bezogen auf das Gewicht dieser Schicht (15), enthält.

18. Tablettenkern, wie beansprucht in den Ansprüchen 1 bis 17, dadurch gekennzeichnet, daß er

   a) den/die wirksame(n) Hauptbestandteil(e),

   b) 5 bis 30 Gew.-% Hydroxypropylmethylcellulose,

   c) bis zu 90 Gew.-% Füllstoff(e),

   d) bis zu 5 Gew.-% (eines) Bindemittel(s) und

   e) bis zu 5 Gew.-% (eines) Gleitmittel(s),
   in dem Einschicht-Tablettenkern, bezogen auf das Gewicht des Tablettenkerns (auf einer nicht-beschichteten Basis), oder, im Falle eines Zweischicht-Tablettenkerns, in der ersten Schicht (14) desselben, d.h. derjenigen mit dem(den) wirksamen Hauptbestandteil(en) [a], bezogen auf das Gewicht dieser Schicht (14), enthält, und ferner der Tablettenkern den Filmüberzug von 3 bis 25 mg/cm$^2$, enthaltend das Ammoniummethacrylat-Copolymer oder ein Gemisch davon, bezogen auf die Tablettenkern-Oberfläche, aufweist, auf welchem Überzug eine oder mehrere Bohrung(en) (13; 16, 17) mit einen Querschnitt von 0,01 bis 1,0 mm$^2$ vorhanden ist/sind.

19. Tablettenkern, wie beansprucht in Anspruch 18, dadurch gekennzeichnet, daß im Falle eines Zweischicht-Tablettenkerns die zweite Schicht (15) davon, welche keinen wirksamen Hauptbestandteil enthält,

a) 30 bis 100 Gew.-% Hydroxypropylmethylcellulose, die eine höhere Viskosität als 1000 cP in 2 Gew.-%iger wäßriger Lösung aufweist,

b) bis zu 70 Gew.-% Füllstoff(e),

c) bis zu 5 Gew.-% (eines) Bindemittel(s) und

d) bis zu 5 Gew.-% (eines) Gleitmittel(s),
bezogen auf das Gesamtgewicht dieser Schicht (15), enthält, und die Bohrung(en) (16; 17) nur auf dem Teil des Filmüberzugs (11), der in Kontakt mit der ersten Schicht (14), enthaltend den wirksame(n) Hauptbestandteil(e), steht [Bohrung 16], vorhanden ist/sind oder, hierüber hinausgehend, auch auf dem Teil des Filmüberzugs (11), der in Kontakt mit der zweiten Schicht (15), die hydrophiles Polymer und keinen wirksamen Hauptbestandteil enthält, steht [Bohrung(en) 17].

20. Verfahren zur Herstellung der Einschicht-Tablettenkerne, wie beansprucht in den Ansprüchen 1 bis 4, 6, 8, 10, 16 oder 18, dadurch gekennzeichnet, daß der(die) therapeutisch wirksame(n) Hauptbestandteil(e) und die Hydroxypropylmethylcellulose gemischt werden, und die unbeschichteten Tablettenkerne (12) durch Kompression hergestellt werden, und letztere (12) mit dem Filmüberzug (11) beschichtet werden, der Ammoniummethacrylat-Copolymer(e) enthält, und schließlich eine oder mehrere Bohrung(en) (13) auf dem Filmüberzug (11) erzeugt wird/werden.

21. Verfahren, wie beansprucht in Anspruch 20, zur Herstellung der Zweischicht-Tablettenkerne, wie beansprucht in den Ansprüchen 1, 2, 5, 7, 17 oder 19, dadurch gekennzeichnet, daß der(die) therapeutisch wirksame(n) Hauptbestandteil(e) und die Hydroxypropylmethylcellulose gemischt werden, und die unbeschichteten Tablettenkerne (12), welche als die erste Tablettenkernschicht (14) mit dem/den wirksamen Hauptbestandteil(en) dienen, durch Kompression hergestellt werden, und entweder darauf die aus Hydroxypropylmethylcellulose bestehende zweite Tablettenkernschicht (15) aufgepreßt wird oder diese zweite Tablettenkernschicht (15) durch Aufpressen eines Pulvers oder Granulats aus Hydroxypropylmethylcellulose auf den als die erste Tablettenkernschicht (14) mit dem/den wirksamen Hauptbestandteil(en) dienenden unbeschichteten Tablettenkern entwickelt wird, und die Oberflächen der so erhaltenen Zweischicht-Tablettenkerne mit dem Ammoniummethacrylat-Copolymer(e) enthaltenden Filmüberzug (11) beschichtet werden, und schließlich eine oder mehrere Bohrung(en) (16) auf dem Filmüberzug (11) auf dem Teil des Filmüberzugs (11), der in Kontakt mit der ersten Tablettenkernschicht (14) steht, die den/die wirksame(n) Hauptbestandteil(e) enthält, erzeugt wird/werden.

22. Verfahren, wie beansprucht in den Ansprüchen 20 oder 21, dadurch gekennzeichnet, daß der(die) therapeutisch wirksame(n) Hauptbestandteil(e) und die Hydroxypropylmethylcellulose mit Füllstoff(en) und/oder Bindemittel(n) zusammengemischt werden.

23. Verfahren, wie beansprucht in den Ansprüchen 20 bis 22, dadurch gekennzeichnet, daß die den/die wirksame(n) Hauptbestandteil(e) enthaltende Mischung und die Hydroxypropylmethylcellulose direkt mit Gleitmittel(n) gemischt wird oder nach Granulieren mit Bindemittel(n).

24. Verfahren, wie beansprucht in den Ansprüchen 20 bis 23, dadurch gekennzeichnet, daß als Pulver oder Granulat aus Hydroxypropylmethylcellulose ein solches verwendet wird, das auch Füllstoff(e) und/oder (ein) Bindemittel und/oder (ein) Gleitmittel enthält.

25. Verfahren, wie beansprucht in den Ansprüchen 20 bis 24, dadurch gekennzeichnet, daß eine oder mehrere Bohrung(en) (17) auch auf dem Teil des Filmüberzugs (11), der in Kontakt mit der zweiten Tablettenkern-Schicht (15) steht, die keinen wirksamen Hauptbestandteil enthält, vorgesehen wird/werden.

26. Verfahren, wie beansprucht in den Ansprüchen 20 bis 25, dadurch gekennzeichnet, daß die Beschichtung des unbeschichteten Tablettenkerns mit einer wäßrigen Dispersion des/der Ammoniummethacrylat-Copolymeren oder einer Lösung mit dem/den Ammoniummethacrylat-Copolymer(en) in Ethanol, Isopropanol oder Aceton oder einer Mischung davon durchgeführt wird.

## Revendications

1. Noyau ( 10) de tablette pharmaceutique a une ou deux couches libérant le remède par commande osmotique de diffusion renfermant un ou plusieurs principes thérapeutiquement actifs ainsi qu'un polymère hydrophilique et ayant pour revêtement une pélicule polymérique (11), dans le cas d'un noyau de tablette à deux couches renfermant le (les) principe (s) actifs et du polymère hydrophilique dans sa première couche (14) et du polymère hydrophilique dans sa seconde couche (15), le noyau ayant de plus au moins un trou (13, 16) sur la pélicule (11) qui est en contact avec le noyau de tablette (10) [dans le cas d'un noyau de tablette à une couche] ou [dans le cas de noyau de tablette à deux couches] sur la partie de la pélicule qui est en contact avec la première couche du noyau de tablette (14) renfermant le (les) principe(s) actifs, la caractéristique du noyau en est que le polymère hydrophilique est constitué d' hydroxypropyle - méthylcellulose et que le matériel de revêtement de la pélicule (11) renferme un copolymère de méthacrylate d'ammonium ou un tel mélange.

2. Noyau de tablette tel que présenté dans la demande 1 dont la caractéristique est donc de renfermer a ou plusieurs trous (17) sur la partie de la pélicule (11) en contact avec la seconde couche (15) du noyau de tablette renfermant du polymère hydrophilique et pas de principe actif.

3. Noyau de tablette tel que présenté dans les demandes 1 ou 2 dont la caractéristique consiste en ce que le (les) trou(s) (13,16,17) est/sont d'une section transversale de 0.01 à 1.0 mm2.

4. Noyau de tablette tel que présenté dans les demandes 1 a 3 dont la caractéristique est de renfermer en tant que polymère hydrophilique une hydroxypropyle - méthylcellulose dont les groupes methoxy en représentent 20 à 30 % du poids et les groupes hydroxypropexy 5 à 12 % ainsi que de détenir une viscosité de 3 à 100,000 centi.poises pour une solution aqueuse de 2 % du poids.

5. Noyau de tablette tel que présenté dans les demandes 1 à 4 dont la caractéristique est que, dans le cas d'un noyau de tablette à deux couches, celui-ci , en tant que polymère hydrophilique, renferme dans la seconde couche (16) du noyau de tablette, qui contient du polymère hydrophilique et pas de principe actif, une hydroxypropyle - méthylcellulose ayant une viscosité dépassant 1 000 centi.poises pour une solution aqueuse de 2% du poids.

6. Noyau de tablette tel que présenté dans les demandes 1 à 5 dont la caractéristique est de renfermer de l' hydroxypropyle - méthylcellulose dont la quantitée représente 5 à 30 % du poids dans le noyau de tablette à une couche selon le poids du noyau de tablette (sur une base sans revêtement), ou, dans le cas d'un noyau de tablette à deux couches, dans la première couche (14) de celui-ci renfermant le (les) principes actifs selon le poids de cette couche (14).

7. Noyau de tablette tel que présenté dans les demandes 1 à 6, dont la caractéristique est de renfermer, dans le cas d'un noyau de tablette à deux couches, de l' hydroxypropyle - méthylcellulose dont la quantité selon le poids de la seconde couche (15) du noyau de tablette ne renfermant pas de principe actif est de 30 à 100 % du poids de cette couche (15).

8. Noyau de tablette tel que présenté dans les demandes 1 à 7 dont la caractéristique est que le (les) copolymère(s) (11) de méthacrylate d'ammonium est/sont tel(s) qu'ils détiennent de l'acrylate d'éthyle, du méthacrylate de méthyle et des unités de méthacrylate de (triméthylammonium) - éthyle.

9. Noyau de tablette tel que présenté dans les demandes 1 à 8 dont la caractéristique est que le(s) copolymère (s) de méthacrylate d'ammonium de la pélicule (11) détien(nen)t un poids moléculaire proportionnel de 100,000 à 200,000 et renferme(nt) 1 partie molaire d'unités d'acrylate d'éthyle, 2 parties molaires d'unités de méthacrylate de méthyle et 0,1 à 0,2 partie molaire d'unités de méthacrylate de ( triméthylammonium).- éthyle.

10. Noyau de tablette tel que présenté dans les demandes 1 à 9 dont la caractéristique est que le mélange de copolymère de méthacrylate d'ammonium de la pélicule (11) est composé

a) d'un copolymère de méthacrylate d'ammonium d'un poids moléculaire proportionnel de 150,000 et renfermant une partie molaire d'acrylate d'éthyle, 2 parties molaires de méthacrylate de méthyle et 0,1 partie molaire de méthacrylate de (triméthylammonium)-éthyle ainsi que

b) d'un copolymère de méthacrylate d'ammonium d'un poids moléculaire proportionnel de 150, 000 et renfermant une partie molaire d'acrylate d'éthyle, 2 parties molaires de méthacrylate de méthyle et 0,2 partie molaire de méthacrylate de (triméthylammonium)-éthyle dans un rapport de proportion de poids allant de 1 : 10 à 10 :1.

**11.** Noyau de tablette tel que présenté dans les demandes 1 à 10 dont la caractéristique est que la pélicule (11) renferme un copolymère de méthacrylate d'ammonium ou un tel mélanged'une quantitée, selon la surface du noyau de tablette, allant de 3 à 25mg/cm2.

**12.** Noyau de tablette tel que présenté dans les demandes de 1 à 11 dont la caractéristique est de renfermer un (des) remplissage(s) et/ou un (des) liant(s) et/ou un(des) lubrifiant(s).

**13.** Noyau de tablette tel que présenté dans les demandes de 1 à 12 dont la caractéristique est le (les) remplissage (s) renferme(nt) de la cellulose, de la cellulose microcristalline, de l'amidon, de la lactose, du mannitol et/ou du phosphate de calcium secondaire.

**14.** Noyau de tablette tel que présenté dans les demandes de 1 à 13 dont la caractéristique est que son (ses) liant(s) renferme (nt) de l'hydroxypropyle - cellulose, de la méthylcellulose, de hydroxypropyle - méthylcellulose, de la polyvinylpyrrolidone, de l'éthylcellulose, de la gélatine, de l'amidon soluble dans l'eau, du glycol de polyoxéthylène, du polyvinylalcool et/ou du polyvinylbutyral.

**15.** Noyau de tablette tel que présenté dans les demandes 1 à 14 dont la caractéristique est que le (les) lubrifiant(s) renferme(nt) du stéarate de magnésium, du talc, de l'acide stéarique, de l'huile de castor hydrogénée et/ou du bioxyde de silicone coloïdal.

**16.** Noyau de la tablette tel que présenté dans les demandes 1 à 15 dont la caractéristique est de contenir

a) un (des) remplissage (s) ne dépassant pas 50% du poids,
b) un (des) liant(s) ne dépassant pas 5% du poids, et/ou
c) un(des) lubrifiant(s) ne dépassant pas 5% du poids
dans le noyau de tablette à une couche selon le poids du noyau de tablette (sur une base sans revêtement), ou, dans le cas d'un noyau de tablette à deux couches, dans la première couche (14) selon le poids de cette couche (14) contenant le (les principe(s) actifs.

**17.** Noyau de tablette tel que présenté dans les demandes 1 à 16 dont la caractéristique est, dans le cas d'un noyau à deux couches de contenir

a) un (des) remplissages ne dépassant pas 70% du poids, et/ou
b) un(des) liant(s) ne dépassant pas 5% du poids
c) un (des) lubrifiant(s) ne dépassant pas 5% du poids
dans la seconde couche (15) selon le poids de cette couche (15) ne renfermant pas de principe actif.

**18.** Noyau de tablette tel que présenté dans les demandes 1 à 17 dont la caractéristique est de contenir

a) Le (les) principe(s) actifis).
b) de la méthylcellulose d hydroxypropyle d'un poids de 5 à 10%.
c) un (des) remplissage(s) ne dépassant pas 90% du poids
d) un (des) liants ne dépassant pas 5% du poids et
e) un (des) lubrifiant(s) ne dépassant pas 5% du poids
dans le noyau de tablette à une couche selon le poids du noyau de tablette ( sur une base sans revêtement), ou, dans le cas d'un noyau de tablette à deux couches, dans la première couche (14) de celui-ci, à savoir celle renfermant le (les) principe(s) actif(s) [a], selon le poids de cette couche (14), et de plus le noyau de tablette détient la pélicule de 3 à 25 mg/cm2 contenant le copolymère de méthacrylate d'ammonium ou un tel mélange selon la surface du noyau de tablette sur le revêtement duquel un ou plusieurs trou(s) (13, 16, 17) de 0.01 à 1.0 mm2 de section transversale est/sont présent(s).

**19.** Noyau de tablette tel que présenté dans la demande 18 dont la caractéristique est que, dans le cas d'un noyau de tablette à deux couches, la seconde couche (15) de celui-ci ne renfermant pas de principe actif contienne

a) de l'hydroxypropyle - méthylcellulose d'un poids de 30 à 100% dont la viscosité dépasse 1 000 centi-poises dans une solution aqueuse d'un poids de 2%.

b) du (des remplissage(s) ne dépassant pas 70%, du poids

c) a (des) liant(s) ne dépassant pas 5% du poids

d) un (des) lubrifiant(s) ne dépassant as 5% du poids.
selon le poids total de cette couche (15), et le (les) trou(s) (16,17) n' est/ ne sont présent(s) que sur la partie de la pélicule étant en contact avec la première couche (14) renfermant le(les principe(s) actif(s) [le (les) trou(s)] ou même au-delà de celle-ci à savoir sur la partie de la pélicule étant en contact avec la seconde couche (15) contenant du polymère hydrophilique et pas de principe actif [le (les) trou(s) 17].

20. Processus de préparation du noyau de tablette à une couche tel que présenté dans les demandes 1 à 4, 6, 8, 10, 16 ou 18 dont la caractéristique est de mélanger le(les) principe(s) thérapeutique actif(s) et l'hydroxypropyle - méthylcellulose et de préparer par compression le noyau de tablette sans revêtement (12), ce dernier (12) est revêtu de la pélicule (11) renfermant du (des) copolymère(s) de méthacrylate d'ammonium et on constate finalement un ou plusieurs trou(s) sur la pélicule (11).

21. Processus tel que présenté dans la demande 20 pour la préparation de noyaux de tablette à deux couches tel que présenté dans les demandes 1, 2, 5, 7, 17 ou 19 dont la caractéristique est de mélanger le (les) principe(s) actif(s) thérapeutique(s) et la hydroxypropyle - méthylcellulose et de préparer par compression le noyau de tablette (12) sans revêtement servant de première couche au noyau de tablette (14) renfermant le(les) principe(s) actif(s) et de presser sur chacun d'eux la seconde couche(15) du noyau de tablette constituée d' hydroxypropyle - méthylcellulose ou de développer la seconde couche (15) du noyau de tablette en pressant sur le noyau de tablette sans revêtement tenant lieu de première couche (14) du noyau de tablette renfermant le principe actif (s) une poudre ou un granulat d' hydroxypropyle - méthylcellulose et de revêtir les surfaces des noyaux de tablette à deux couches ainsi obtenues de la pélicule (11) renfermant du (des) copolymère(s) de méthacrylate d'ammonium et de constater finalement un ou plusieurs trou(s) (16) sur la pélicule(11) sur cette partie de la pélicule (11) étant en contact avec la première couche(14) du noyau de tablette renfermant le(les) principe(s) actif(s).

22. Processus tel que demandé dans les demandes 20 ou 21 se caractérise du fait que le (les) principes thérapeutiquement actifs et la méthylcellulose d'hydroxypropyle se trouvent mélangés au(x) remplissage et/ou au(x) liant(s).

23. Processus tel que présenté dans les demandes 20 à 22 se caractérise du fait que le mélange renfermant [e(les) principe(s) actifs et la méthylcellulose d'hydroxypropyle se trouve(nt mélangé(s) au (x) lubrifiant(s) directement ou après granulation avec le (les) liant(s).

24. Processus tel que présenté dans les demandes 20 à 23 dont la caractéristique est d'utiliser poudre ou granule de méthylcellulose d'hydroxypropyle renfermant donc le (les remplissage(s) et/ou le (les) liant(s) et/ou le (les) lubrifiants.

25. Processus tel que présenté dans les demandes 20 à 24 dont la caractéristique est de constater un ou plusieurs trou(s) (17) sur la partie de la pélicule (11) en contact avec la seconde couche (15) du noyau de tablette ne renfermant pas de principe actif.

26. Processus tel que présenté dans les demandes 20 à 25 dont la caractéristique est d'obtenir le revêtement du noyau de tablette sans revêtement par dispersion aqueuse du (des) copolymères de méthacrylate d'ammonium ou à l'aide d'une solution renfermant le(les copolymère(s) de méthacrylate d'ammonium dans de l'éthanol, de l'isopropol ou de l'acétone ou bien d'un mélange de ceux-ci.

Fig. 1                    Fig. 2

16

11

14

10

15

Fig. 3                    Fig. 4

Fig. 5          Fig. 6

Fig. 7

Active principle release of a Salbutamol composition

Time in hours

Released active principle in % by weight

Example 1/I (unbored)

Example 1/I (bored)

Example 1/II (unbored)

Example 1/II (bored)

Active principle release of a Nifedipine composition

Fig. 8

EP 0 662 321 B1

Active principle release of a Nifedipin composition

Fig. 9

EP 0 662 321 B1